(19) Europäisches Patentamt
European Patent Office
Office européen des brevets

(11) Numéro de publication: **0 153 538**
**B1**

(12) # FASCICULE DE BREVET EUROPÉEN

(45) Date de publication du fascicule du brevet: **26.11.86**

(51) Int. Cl.⁴: **C 07 C 69/732**, A 61 K 31/215 // C07C47/575, C07C45/45, C07C67/343, C07C67/303

(21) Numéro de dépôt: **84400361.6**

(22) Date de dépôt: **23.02.84**

(54) **Nouveaux dérivés de l'acide mélilotique, et médicaments les contenant.**

(43) Date de publication de la demande: **04.09.85 Bulletin 85/36**

(45) Mention de la délivrance du brevet: **26.11.86 Bulletin 86/48**

(84) Etats contractants désignés: **AT BE CH DE FR GB IT LI NL**

(56) Documents cité:
**FR-A-2 473 039**
**US-A-2 995 598**

(73) Titulaire: **Laboratoire Marcel Richard Société anonyme dite, Sauzet, F-26200 Montélimar (FR)**

(72) Inventeur: **Richard, Marcel, 12, Rue Saladin, F-07200 Aubenas (FR)**

(74) Mandataire: **Doan, Dinh- Nam, Cabinet BEAU DE LOMENIE 55, rue d'Amsterdam, F-75008 Paris (FR)**

EP 0 153 538 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne les dérivés de l'acide mélilotique répondant à la formule générale:

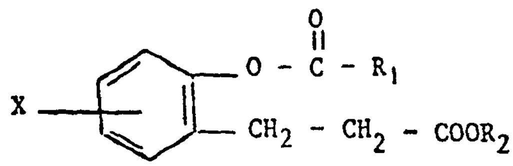

(I)

dans laquelle:

- X représente un atome d'halogène, notamment Cl ou F,
- $R_1$ est un radical alkyle inférieur contenant 1 à 4 atomes de carbone, notamment méthyle,
- $R_2$ est un atome d'hydrogène, un métal notamment un métal alcalin (sodium, potassium) ou alcalino terreux (calciut) ou un radical ammonium ou un radical alkyle inférieur de 1 à 4 atomes de carbone.

Plus particulièrement, la présente invention concerne les dérivés monohalogénés de l'acide acétyl mélilotique (ou acide acétoxy-2 phényl propionique) de formule générale:

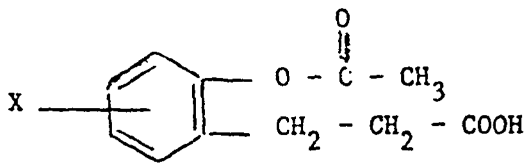

(II)

dans laquelle X est un atome d'halogène, notamment Cl ou F, ainsi que les sels notamment les sels de métaux alcalins, alcalino terreux ou d'ammonium et les esters desdits dérivés.

Plus particulièrement encore, l'invention a pour objet l'acide fluoro-5 acétyl mélilotique (ou acide fluoro-5 acétoxy-2 phényl propionique) de formule

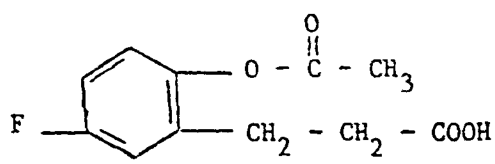

et ses sels notamment ses sels de métaux alcalin, ou alcalino terreux ou d'ammonium et ses esters.

Les composés selon l'invention présentent des propriétés antiagrégantes plaquettaires, cardiovasculaires et analgésiques remarquables comparativement aux composés non halogénés correspondants.

Ils sont de ce fait utiles comme médicaments à usage humain et vétérinaire pour le traitement:

- des affections cardiovasculaires,
- de vasodilatation périphérique,
- des affections circulatoires,
- de l'ischemie des membres,
- des artérites

et également pour la prévention et le traitement des troubles plaquettaires et pour le traitement des algies.

Plus particulièrement, l'acide fluoro-5 acétyl mélilotique se présente sous la forme d'une poudre cristalline blanche, peu soluble dans l'eau, mais soluble dans l'alcool, l'acétone et dans les solvants organiques usuels. Son point de fusion est de 96-98° C.

Sa toxicité est de 1 500 mg/kg par voie orale et de 750 mg/Kg par voie intrapéritonéale, déterminée sur la souris mâle de la souche SWISS.

Il présente une activité remarquable en ce qui concerne l'effet antiagrégant plaquettaire et anti-inflammatoire vasculaire; il améliore la circulation de retour, protège les parois vasculaires, prévient les thromboses intravasculaires et renforce la résistance capillaire.

Les propriétés pharmacologiques de ce produit ont été mises en évidence dans les essais sur le rat, la souris et le chien.

Ce produit inhibe l'agrégation plaquettaire provoquée par l'ADP (adénosine diphosphate)

sur le sang humain, inhibition de 40 % à 100 mg/Kg,

sur le rat et le lapin, inhibition de 60 % à 100 mg/Kg.

Sur le rat anesthésié à l'éthylméthane, on enregistre après administration I.V. de 100 mg/Kg de produit selon l'invention une légère hypotension, soit une baisse de 15%.

Chez le chien anesthésié au Pentobarbital, on enregistre après injection IV. de 50 mg/Kg de produit selon l'invention une hypotension de 7 %, une augmentation du débit de 65 %, une diminution du rythme respiratoire

de 25 % et un renforcement des battements sans modification de la fréquence cardiaque.

En ce qui concerne l'activité analgésique, le produit donne dans le test à la phénylbenzoquinone et dans le test à la plaque chauffante chez la souris une protection de 88 % à 500 mg/Kg.

Comme exemple d'application thérapeutique, le produit a donné des résultats favorables en artériopathies périphériques, et dans le traitement de l'oedème de la cheville, et des varices.

Les préparations pharmaceutiques contenant comme ingrédient actif un composé selon l'invention associé à un support ou véhicule non toxique pharmaceutiquement acceptable peuvent être présentées sous les formes de dosage unitaires suivantes:

- pour l'administration interne:

ampoules injectables et buvables, gélules, comprimés, poudres, granulés, suppositoires (substance pure ou associée à d'autressubstances médicamenteuses) dosés à 0,25 g

- pour l'administration externe:

crème, pommades, solutés, émulsion (substance pure ou associée à d'autres substances médicamenteuses).

On peut préparer les composés selon la présente invention de formules I et II par un procédé comprenant les étapes suivantes:

a) - réaction d'un halogénophénol avec du chloroforme et un alcali, tel que la potasse, pour former un aldéhyde d'halogénophénol (selon la réaction de Reimer-Tiemann),

b)- condensation de cet aldéhyde avec l'anhydride acétique en presence d'acétate de potassium pour former le dérivé halogéné de l'acide acétoxy-2 cinnamique (selon la réaction de perkin),

c) - hydrogénation catalytique de l'acide obtenu pour former le dérivé halogéné de l'acide acétyl mélilotique.

On peut préparer les sels de ce dernier par réaction de celuici avec une base et les esters par réaction avec un alcool.

Plus particulièrement, on peut préparer l'acide fluoro-5 acétyl mélilotique par les etapes suivantes:

a) - réaction du para-fluorophénol avec du chloroforme et de lapotasse pour former l'aldéhyde fluoro-5 salicylique,

b) - condensation de cet aldéhyde avec l'anhydride acétique en présence d'acétate de potassium pour former l'acide fluoro-5 acétoxy-2 cinnamique,

c) - hydrogénation de ce dernier par de l'hydrogène en présence d'un catalyseur au palladium sur support de charbon pour former l'acide fluoro-5 acétyl mélilotique:

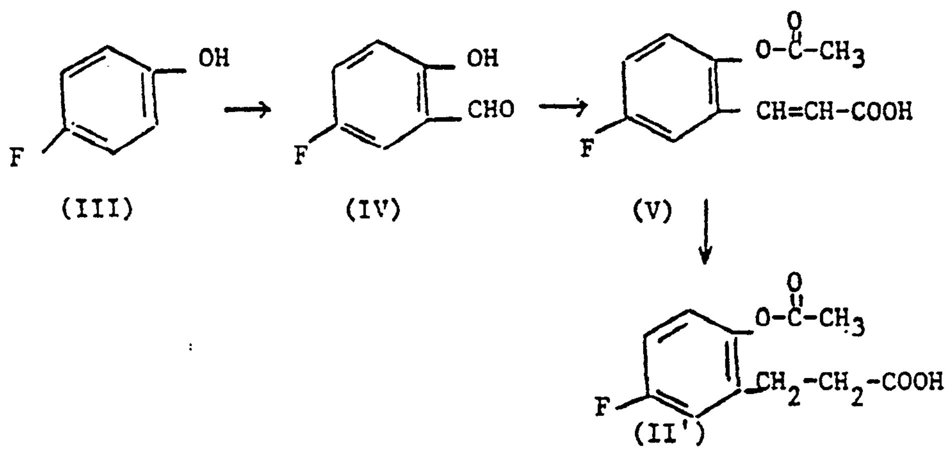

**Exemple**

- Préparation de l'acide fluoro-5 acétyl mélilotique

a) Aldéhyde fluoro-5 salicylique (IV)

- Dans un ballon muni d'un agitateur, on prépare une solution de 240 g (6 moles) de soude en pastilles avec 250 $cm^3$ d'eau distillée. Dès que la température se stabilise vers 60- 65°C, on ajoute par portions, avec agitation, 55 g (0,5 mole) de parafluorophénol. On chauffe le mélange au bain-tarie avec réfrigérant ascendant de façon à ce que la température interne soit de 70°C.

On ajoute, par le réfrigérant, 118 g (80 $cm^3$) de chloroforme par petites portions.

On laisse au repos 1 heure à 70°C, puis on entraine à la vapeur l'excès de chloroforme.

Lorsque tout le chloroforme en excès est passé, on acidifie avec beaucoup de précaution le mélange encore

tiède à l'acide sulfurique à 50% en le refroidissant éventuellement par des glaçons. Le pH du mélange est d'environ 2.

On entraîne à la vapeur l'aldéhyde. Après 12 heures de repos à 0°C, on essore, lave avec de l'eau glacée et sèche à l'air.

Rendement: 17 g d'aldéhyde fluoro-5 salicylique sous forme de gros cristaux jaunâtres, d'odeur irritante.

P.F. = 72 - 74°C (Tube capillaire, non corrigé).

Analyse pour $C_7H_5FO_2$ (pM = 140)
Calculé C% 60,00 H% 3,57
Trouvé C% 59,85 H% 3,63

Spectre infra-rouge
CO à 1660 $cm^{-1}$
OH lié à 3100 cm

b) Acide fluoro-5 acétoxy-2-cinnamique (V)

22 g (0,13 mole) de l'aldéhyde obtenu ci-dessus
30 $cm^3$ d'anhydride acétique,
et 20 g d'acétate de potassium, fraichement fondu, sont chauffés dans un ballon muni d'un tube de dégagement à 180°C au bain de sable, pendant 2 heures.

On refroidit à 60°C, on ajoute de l'eau, et on entraîne à la vapeur l'aldéhyde en excès. La solution restante est décolorée si nécessaire par du charbon actif. On filtre la solution bouillante.

- Les cristaux qui se séparent sont un mélange de dérivés hydroxy-2 et acétoxy-2.

Après sèchage en dessicateur, on acétyle par un mélange pyridineanhydride acétique pendant 24 heures à la température ambiante. Après évaporation sous vide, le résidu est repris par l'acétate d'éthyle à l'acide HCl dilué, à l'eau, et séché sur $SO_4Na_2$ anhydre.

Après filtration et évaporation sous vide, on obtient l'acide fluoro-5 acétoxy-2 cinnamique pur sous forme cristallisée.

- Rendement 12 g PF: 194° - 196°C

Analyse pour $C_{11}H_9O_4F$ (PM = 244)
Calculé C % 58,92 H % 4,08
Trouvé C % 58,95 H % 3,82

Spectre infra-rouge: CO acide à 3000 $cm^{-1}$
CO acétate à 1760 $cm^{-1}$
ester à 1220 $cm^{-1}$ c) Hydrogénation

12 g d'acide fluoro-5 acétoxy-2 cinnamique sont dissous dans 120$cm^3$ acétate d'éthyle avec 1,5 g de palladium sur charbon à 10 % et hydrogénés dans l'appareil de Parr sous 5 atmosphères d'hydrogène pendant 1 heure; on filtre et concentre sous vide pour obtenir un résidu cristallisé d'acide fluoro-5 acétylmélilotique pur peu soluble dans l'eau, soluble dans l'alcool, l'acétone et les solvants organiques usuels.

PF: 96° - 98°C

Chromatographie en couche mince (Chloroforme-méthanol à 5%) Rf = 0,8 (faiblement fluorescent)

Analyse pour $C_{11}H_{11}O_4F$ (PM 226)
Calculé C % 58,40 H % 4,86
Trouvé C % 58,52 H % 5,00

## Revendications

pour les Etats contractants BE CH DE FR GB IT LI NL

1. Nouveaux dérivés de l'acide mélilotique répondant à la formule générale:

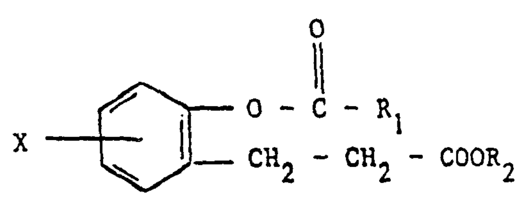

(I)

dans laquelle:

- X représente un atome d'halogène,
- $R_1$ est un radical alkyle inférieur contenant 1 à 4 atomes de carbone,
- $R_2$ est un atome d'hydrogène, un métal ou un radical ammonium ou un radical alkyle inférieur de 1 à 4 atomes de carbone.

2. Dérivés monohalogénés de l'acide acétyl mélilotique de formule générale:

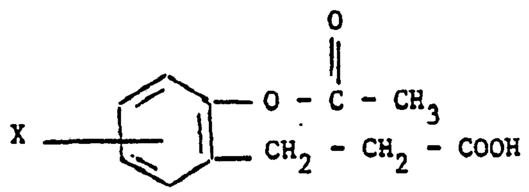

(II)

dans laquelle X est un atome d'halogène, ainsi que les sels et esters d'alkyle de 1 à 4 atomes de carbone desdits dérivés.

3. Acide fluoro-5 acétyl mélilotique

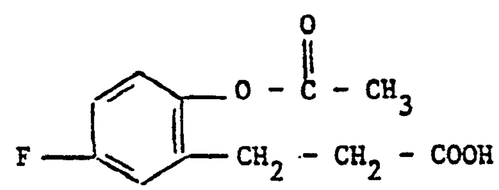

(III)

et ses sels et esters d'alkyle de 1 à 4 atomes de carbone.

4. Dérivés selon la revendication 1 ou 2, caractérisés en ce que X représente un atome de fluor ou de chlore.

5. Dérivés selon la revendication 1 ou 2 ou 3, caractérisés en ce que le sel est un sel d'un métal alcalin ou alcalino terreux ou un sel d'ammonium.

6. Médicament à usage humain ou vétérinaire, caractérisé en ce qu'il comprend un composé selon l'une des revendications 1 à 5.

7. Composition pharmaceutique caractérisée en ce qu'elle contient comme ingrédient actif un médicament selon la revendication 6, associé au poids médicinal à un support ou véhicule non toxique, pharmaceutiquement acceptable.

8. Composition pharmaceutique selon la revendication 7 caractérisée en ce qu'elle contient comme ingrédient actif l'acide fluoro-5 acétyl mélilotique.

9. Composition pharmaceutique selon la revendication 8, caractérisée en ce qu'elle est présentée sous forme de dosage unitaire pour l'administration par voie orale, anale ou parentérale.

**Revendications**

pour l'Etat contractant AT.

1. Procédé de préparation des dérivés de l'acide mélilotique répondant à la formule générale:

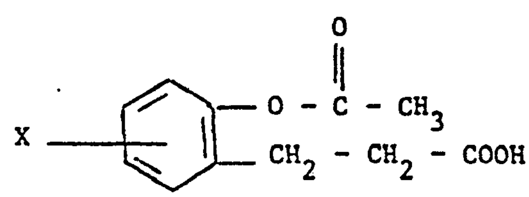

dans laquelle X est un atome d'halogène, notamment Cl ou F, ainsi que les sels notamment les sels de métaux alcalins, alcalino terreux ou d'ammonium et les esters d'alkyle de 1 à 4 atomes de carbone lesdits dérivés, caractérisé en ce qu'il comprend les étapes suivantes:

a) réaction d'un halogénophénol avec du chloroforme et un alcali, pour former un aldéhyde d'halogénophénol selon la réaction de Reimer-Tiemann,

b) condensation de cet aldéhyde avec l'anhydride acétique en présence d'acétate de potassium pour former le dérivé halogéné de l'acide acétoxy-2 cinnamique selon la réaction de Perkin,

c) hydrogénation catalytique de l'acide obtenu pour former le dérivé halogéné de l'acide acétylmélilotique,

d) éventuellement transformation de cet acide en un sel ou en un ester de manière connue en soi.

2. Procédé de préparation de l'acide fluoro-5 acétylmélilotique de formule

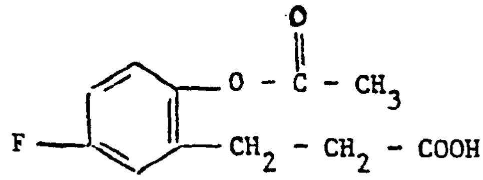

et ses sels notamment ses sels de métaux alcalins,ou alcalino terreux ou d'ammonium et ses esters d'alkyle de 1 à 4 atomes de carbone caractérisé en ce qu'il comprend les étapes suivantes:

a) réaction du para-fluorophénol avec du chloroforme et de la potasse pour former l'aldéhyde fluoro-5 salicylique,

b) condensation de cet aldéhyde avec l'anhydride acétique en présence d'acétate de potassium pour former l'acide fluoro-5 acétoxy-2 cinnamique,

c) hydrogénation de ce dernier par de l'hydrogène en présence d'un catalyseur au palladium sur support de charbon pour former l'acide fluoro-5 acétyl mélilotique,

d) éventuellement transformation de l'acide en un sel ou en un ester de manière connue en soi.

3. Procédé de préparation d'une cotposition pharmaceutique à usage humain ou vétérinaire notamment à effet anti-agrégant plaquettaire et anti-inflammatoire vasculaire, caractérisé en ce que l'on associe un dérivé de l'acide mélilotique obtenu selon l'une des revendications 1 à 2 ou un sel ou ester dudit dérivé avec un support ou véhicule non toxique, pharmaceutiquement acceptable et que l'on met cette association sous la forme de doses unitaires.

4. Procédé selon la revendication 3 caractérisé en ce que le dérivé de l'acide mélilotique est l'acide fluoro-5 acétyl mélilotique.

**Patentansprüche**

für die Vertragsstaaten BE, CH, DE, FR, GB, IT, LI, NL:

1. Neue Melilotinsäurederivate der allgemeinen Formel

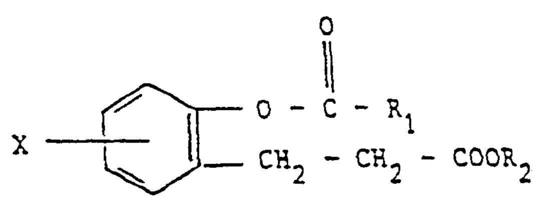

(I)

worin:

- X ein Halogenatom darstellt,
- $R_1$ eine Niedrigalkylgruppe mit 1 bis 4 Kohlenstoffatomen ist,
- $R_2$ ein Wasserstoffatom, ein Metall oder einen Ammoniumrest oder eine Niedrigalkylgruppe mit 1 bis 4 Kohlenstoffatomen bedeutet.

2. Monohalogenierte Melilotinsäurederivate der allgemeinen Formel

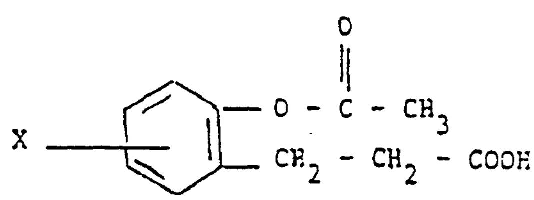

(II)

worin X ein Halogenatom darstellt, sowie die Salze und $C_1$-$C_4$-Alkylester dieser Derivate.

3. 5-Fluor-acetyl-melilotinsäure

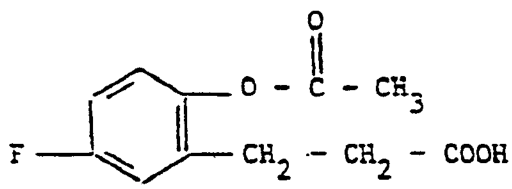

(III)

und ihre Salze und $C_1$-$C_4$-Alkylester.

4. Derivate nach Anspruch 1 oder 2, dadurch gekennzeichnet, daß X ein Fluor- oder Chloratom darstellt.

5. Derivate nach Anspruch 1 oder 2 oder 3, dadurch gekennzeichnet, daß das Salz ein Alkalimetall- oder Erdalkalimetallsalz oder ein Ammoniumsalz ist.

6. Medikamente zur Verwendung in der Human- oder Veterinärmedizin, dadurch gekennzeichnet, daß es eine Verbindung nach einem der Ansprüche 1 bis 5 umfaßt.

7. Pharmazeutische Zusammensetzung, dadurch gekennzeichnet, daß sie als aktives Ingrediens ein Medikament nach Anspruch 6 in Verbindung mit einem nichttoxischen, pharmazeutisch akzeptablen Träger oder Vehikel in medizinisch wirksamer Menge enthält.

8. Pharmazeutische Zusammensetzung nach Anspruch 7, dadurch gekennzeichnet, daß sie als aktives Ingrediens 5-Fluor-acetyl-melilotinsäure enthält.

9. Pharmazeutische Zusammensetzung nach Anspruch 8, dadurch gekennzeichnet, daß sie in Einheitsdosisform für Verabreichung auf oralem, analem oder parenteralem Weg vorliegt.

**Patentansprüche**

für den Vertragsstaat AT:

1. Verfahren zur Herstellung von Melilotinsäurederivaten der allgemeinen Formel

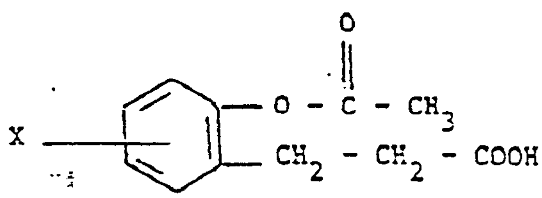

worin X ein Halogenatom, insbesondere Cl oder F, darstellt, sowie der Salze, insbesondere der Alkalimetall-, Erdalkali-Ammoniumsalze und der $C_1$-$C_4$-Alkylester dieser Derivate, dadurch gekennzeichnet, daß es die folgenden Schritte umfaßt:

a) Umsetzung eines Halogenphenols mit Chloroform und einem Alkali zur Bildung eines Halogenphenolaldehyds nach der Reimer-Tiemann-Reaktion,

b) Kondensation dieses Aldehyds mit Essigsäureanhydrid in Gegenwart von Kaliumacetat zur Bildung des halogenierten Derivats der 2-Acetoxyzimtsäure nach der Perkin-Reaktion,

c) katalytische Hydrierung der erhaltenen Säure zur Bildung des halogenierten Acetylmelilotinsäurederivats,

d) gegebenenfalls Umwandlung dieser Säure in ein Salz oder einen Ester auf an sich bekannte Weise.

2. Verfahren zur Herstellung von 5-Fluor-acetyl-melilo-tinsäure der Formel

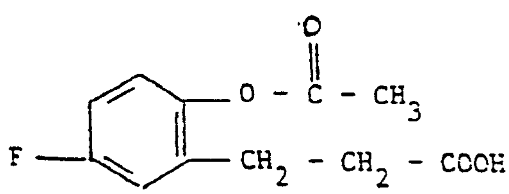

und ihrer Salze, insbesondere der Alkalimetall- oder Erdalkalimetall- oder Ammoniumsalze und ihrer $C_1$-$C_4$-Alkylester, dadurch gekennzeichnet, daß es die folgenden Schritte umfaßt:

a) Reaktion von para-Fluorphenol mit Chloroform und Kaliumlauge zur Bildung von 5-Fluor-salicylaldehyd,

b) Kondensation dieses Aldehyds mit Essigsäureanhydrid in Gegenwart von Kaliumacetat zur Bildung von 5-Fluor-2-acetoxy-zimtsäure,

c) Hydrierung letzterer mit Wasserstoff in Gegenwart eines Palladiumkatalysators auf Kohleträger zur Bildung der 5-Fluor-acetyl-melilotinsäure,

d) gegebenenfalls Umwandlung der Säure in ein Salz oder einen Ester auf an sich bekannte Weise.

3. Verfahren zur Herstellung einer pharmazeutischen Zusammensetzung zur Verwendung in der Human- oder Veterinärmedizin, insbesondere mit plättchenaggregationshemmender und gefäßentzündungshemmender Wirkung, dadurch gekennzeichnet, daß man ein nach einem der Ansprüche 1 bis 2 erhaltenes Melilotinsäurederivat oder ein Salz oder einen Ester dieses Derivats mit einem nichttoxischen, pharmazeutisch akzeptablen Träger oder Vehikel vereinigt und diese Kombination in Einheitsdosisform bringt.

4. Verfahren nach Anspruch 3, dadurch gekennzeichnet, daß das Melilotinsäurederivat 5-Fluor-acetyl-melilotinsäureacetyl ist.

**Claims**

For the contracting states BE, CH, DE, FR, GB, IT, LI, NL

1. New melilotic acid derivatives of the general formula:

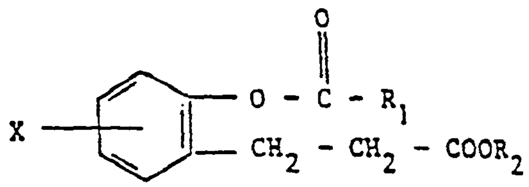

(I)

wherein

- X represents a halogen atom
- $R_1$ is a lower alkyl radical of 1 to 4 carbon atoms,
- $R_2$ is a hydrogen atom, a metal an ammonium radical or a lower alkyl radical of 1 to 4 carbon atoms.

2. Monohalogenated derivatives of acetylmelilotic acid of the general formula:

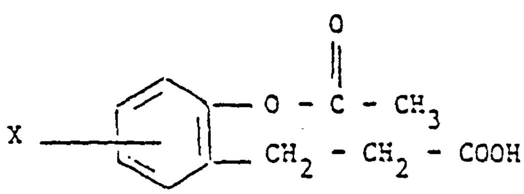

(II)

wherein X is a halogen atom, as well as salts and alkyl (of 1 to 4 carbon atoms) esters of said derivatives.

3. 5-fluoro acetylmelilotic acid

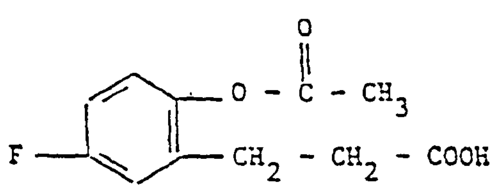

(III)

and salts and alkyl (of 1 to 4 carbonatoms) esters of said acid.

4. Derivatives according to claim 1 or 2, charaterized in that X represents fluorine or chlorine atom.

5. Derivatives according to claim 1 or 2 or 3, characterized in that the salt is an alkali metal salt, or an alkaline-earth metal salt or an ammonium salt.

6. Medicament for human or veterinary use characterized in that it comprises a compound according to any one of claims 1 to 5.

7. Pharmaceutical composition characterized in that it contains as an active ingredient a medicament according to claim 6, associated in an active amount to a non-toxic pharmaceutically acceptable carrier or vehicle.

8. Pharmaceutical composition according to claim 7 characterized in that it contains as an active ingredient 5-fluoro acetylmelilotic acid.

9. Pharmaceutical composition according to claim 8, characterized in that it is presented in the form of unitary dosage for oral, anal or parenteral administration.

**Claims**

for the contracting State AT.

1. Process for preparing melilotic acid derivatives of the general formula:

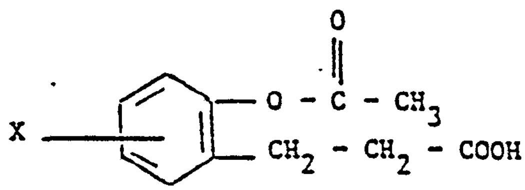

wherein:

X is a halogen atom, specially Cl or F, as well as salts, specially alkali metal salts, alkaline-earth metal salts, or ammonium salts, and alkyl (of 1 to 4 carbon atoms) esters of said derivatives, characterized in that it comprises the following steps:

a) reaction of a halogenophenol with chloroform and an alkali so as to form a halogenophenol aldehyde according to Reimer-Tiemann reaction,

b) condensation of said aldehyde with acetic acid anhydride in the presence of potassium acetate so as to form a halogenated derivative of 2-acetoxy cinnamic acid according to Perkin reaction,

c) catalytic hydrogenation of the obtained acid so as to form a halogenated derivative of acetylmelilotic acid,

d) optionally transformation of said acid into a salt or an ester in a known manner.

2. Process for preparing 5-fluoro acetylmelilotic acid of the formula:

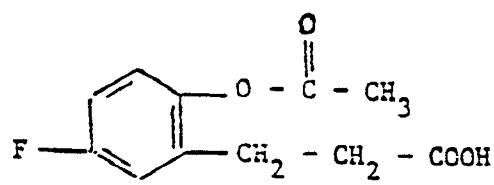

and its salts, specially alkali metal salts, or alkaline-earth metal salts or ammonium salts, and its alkyl (of 1 to 4 carbon atoms) esters, characterized in that it comprises the following steps:

a) reaction of para-fluorophenol with chloroform and potassium hydroxide to form 5-fluoro salicylaldehyde,

b) condensation of said aldehyde with acetic acid anhydride in the presence of potassium acetate to form 5-fluoro 2-acetoxy cinnamic acid,

c) hydrogenation of the latter by hydrogen in the presence of a palladium on charcoal catalyst to form 5-fluoro acetylmelilotic acid,

d) optionally transformation of said acid into a salt or an ester in a known manner.

3. Process for preparing a pharmaceutical composition for human or veterinary use, specially for its blood platelet aggregation inhibitor action and its vascularanti-inflammatory action, characterized in that a melilotic acid derivative obtained according to any of claims 1 and 2, or a salt or an ester of said derivative, is associated with a non-toxic, pharmaceutically acceptable carrier or vehicle and said association is put in the form of unitary dosages.

4. Process according to claim 3 characterized in that melilotic acid derivative is 5-fluoro acetylmelilotic acid.